(19) Europäisches Patentamt
European Patent Office
Office européen des brevets

(11) Publication number: 0 189 587 B1

(12) EUROPEAN PATENT SPECIFICATION

(45) Date of publication of patent specification: 13.12.89

(51) Int. Cl.[4]: A 61 L 2/18, C 12 P 21/02, A 01 N 47/44

(21) Application number: 85116457.4

(22) Date of filing: 23.12.85

# (54) Method of sterilizing recombinant microorganism.

(30) Priority: 27.12.84 JP 281377/84

(43) Date of publication of application:
06.08.86 Bulletin 86/32

(45) Publication of the grant of the patent:
13.12.89 Bulletin 89/50

(84) Designated Contracting States:
AT BE CH DE FR GB IT LI LU NL SE

(56) References cited:
CHEMICAL ABSTRACTS, vol. 93, no. 11, 15th September 1980, page 110, abstract no. 107920x, Columbus, Ohio, US; R.M.M. KLEMPERER et al.: "Effect of R-plasmid RP1 and nutrient depletion on the resistance of Escherichia coli to cetrimide, chlorhexidine and phenol", & J. APPL. BACTERIOL. 1980, 48(3), 349-57

(73) Proprietor: Suntory Limited
1-40 Dojimahama 2-chome Kita-ku
Osaka-shi Osaka-fu 530 (JP)

(72) Inventor: Sugimoto, Shunjirou Institute Biomedical Research
Suntory Limited 1-1, Wakayamadai, 1-chome
Shimamoto-cho Mishima-gun Osaka (JP)
Inventor: Sugimura, Keijirou Institute Biomedical Research
Suntory Limited 1-1, Wakayamadai, 1-chome
Shimamoto-cho Mishima-gun Osaka (JP)
Inventor: Sawano, Toshihiro Institute Biomedical Research
Suntory Limited 1-1, Wakayamadai, 1-chome
Shimamoto-cho Mishima-gun Osaka (JP)
Inventor: Higashi, Naoki Institute Biomedical Research
Suntory Limited 1-1, Wakayamadai, 1-chome
Shimamoto-cho Mishima-gun Osaka (JP)

(74) Representative: Lehn, Werner, Dipl.-Ing. et al
Hoffmann, Eitle & Partner Patentanwälte
Arabellastrasse 4
D-8000 München 81 (DE)

EP 0 189 587 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a method of sterilizing recombinant microorganisms. More particularly, the present invention relates to a method of sterilizing the cultured cells of a physiologically active substance producing recombinant microorganism without impairing the physicochemical or physiological properties of said substance.

The term recombinant microorganism used hereinafter means a microorganism that has the ability to produce a physiologically active polypeptide and which has been transformed by a plasmid or a phage vector constructed by the recombinant DNA technology or gene manipulation technology.

When a recombinant microorganism is cultured and the useful substance produced from said microorganism is extracted and purified from the culture, it is required that the recombinant microorganism should not leak to the environment for the purposes of preventing regrowth of the microorganism and complying with the guidelines for experiments with recombinant DNA. This requirement could be met by containing all steps ranging from the cultivation to purification within sealed conditions, but this is not an easy job to do and, instead, the guidelines call for employing a certain method in order to completely sterilize the cultured recombinant microorganism before it is subjected to extracting and purifying steps.

Microorganisms are conventionally sterilized either by physical methods using heating or disruption of organism cells, or by chemical methods using chlorine based sterilizers, xylene based sterilizers or hydrogen peroxide. However, when one of these methods is used to sterilize a certain microorganism which is intended to be treated for the purpose of extracting and purifying a useful substance from the microorganism per se or a culture thereof, the inherent properties of the desired substance may be impaired by the sterilizing effect of the method. Further problems result from the use of sterilizing chemicals; chlorine-based sterilizers and hydrogen peroxide will corrode the cultivation vessel or tank, or pipes and valves in the cultivation system, whereas xylene-based chemicals have the potential problem of explosion. These problems render the use of sterilizing chemicals particularly unsuitable for the purpose of sterilizing large cultures of recombinant microorganism.

The present inventors made various studies with a view to solving the aforementioned problems and have found that they can be solved by employing as a sterilizing agent an aqueous solution containing chlorhexidine gluconate. The present invention has been accomplished on the basis of this finding.

The following description of the present invention concerns the use of an aqueous solution of chlorhexidine gluconate as a sterilizing agent, but it should be understood that any solution, for example, an ethanol solution may be used if it contains the above salt as the principal component. If desired, a surfactant such as an emulgen-based surfactant may be incorporated in the solution of chlorhexidine gluconate.

Chlorhexidine, or 1,6-di(4′-chlorophenylguanido)-hexane, has the following structure:

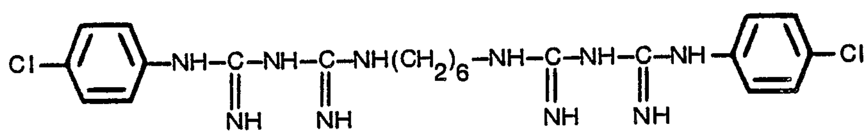

and low concentrations of this compound will exhibit rapid sterilizing action on a broad spectrum of microorganisms, especially bacteria. A gluconic acid salt of chlorhexidine is extensively used as a sterilizer or disinfectant applied either to human beings externally or to medical equipment.

In accordance with the present invention, a low concentration of chlorhexidine gluconate is added to a liquid culture of a recombinant microorganism so as to kill the microorganism without impairing the physicochemical or physiological properties of the useful substance produced within the microorganism cells. The chlorhexidine gluconate is customarily used as a sterilizer or disinfectant applied either to human beings externally or to medical equipment, but in no case has it been used as a sterilizer for a culture of microorganisms, to say nothing of a recombinant microorganism capable of producing a useful substance. The gluconic acid salt of chlorhexidine has high solubility in water, is simple to use and will present minimum hazards to the user because of its relatively low toxicity to humans. Therefore, the gluconic acid salt of chlorhexidine is particularly useful when a large culture of a recombinant microorganism capable of producing a useful substance, must be sterilized.

Chlorhexidine gluconate is preferably used as an aqueous solution. If the useful substance produced within the cells of the microorganism is to be extracted therefrom simultaneously with sterilization, an aqueous solution containing not only chlorhexidine gluconate but also a surfactant such as an emulgen-based surfactant may be used advantageously.

The concentration of chlorhexidine gluconate that is capable of sterilizing a recombinant microorganism without impairing any of the properties of the useful substance produced by said microorganism will vary depending upon the species or strain of the recombinant microorganism, its cultivation concentration or the type of the culture medium, but preferably the final concentration of chlorhexidine gluconate ranges from 0.03% to 0.5%. Stated more specifically, a 20% aqueous solution of chlorhexidine gluconate is added to a liquid culture of recombinant microorganism in such an amount that the concentration of chlorhexidine gluconate will lie between 0.03% and 0.5% and the resulting mixture is stirred for a suitable period that generally ranges from 30 minutes to 6 hours. The temperature at which the culture is treated with chlorhexidine gluconate is not limited to any particular value but any extreme temperatures that may impair the properties of the useful substance produced by the microorganism should be avoided.

The so sterilized liquid culture of recombinant microorganism is then centrifuged to collect dead cells, which are disrupted by appropriate means and transferred to suitable steps of extracting and purifying the desired useful substance from the cells. The chlorhexidine gluconate that has been added as a sterilizer may be readily removed in the purifying process. As will be described more specifically in the following examples and reference example, a useful physiologically active polypeptide (i.e., polypeptide having the activity of human interferon) can be extracted and purified, without any damage to its physicochemical or physiological properties, from the recombinant microorganism (*E. coli*) sterilized by the method of the present invention.

The following examples and reference example assume the use as a recombinant microorganism of a transformed *E. coli* having the ability to produce a polypeptide having the human interferon activity, but it should be understood that the sterilizing method of the present invention is applicable to microorganisms other than *E. coli*. It should also be understood that the range of useful substances that can be extracted and purified from recombinant microorganisms is not limited to the polypeptide having human interferon activity.

## Example 1

Concentration-Dependent Sterilizing Effect of Chlorhexidine Gluconate:

A recombinant microorganism *E. coli* W3110/pIN5T4 (disclosed in Japanese Patent Application No. 132524/1983) having the ability to produce a polypeptide having the human gamma interferon activity was cultivated under aeration and agitation at 30°C for 36 hours in a medium containing 3% polypeptone, 2% yeast extract, 2% glucose, 0.5% $KH_2PO_4$, 0.01% $MgSO_4 \cdot 7H_2O$ and 20 µg/ml of tetracycline. The resulting liquid culture contained $3.2 \times 10^7$ CFU (colony forming units) of viable *E. coli* cells per ml. To this liquid culture, an aqueous solution of 20% chlorhexidine gluconate was added in varying amounts ranging from 0.03% to 0.18% in terms of chlorhexidine gluconate. After agitation at 30°C for 1 hour, the number of viable *E. coli* cells in the culture was counted. The results are shown in Table 1, from which one can see that chlorhexidine gluconate was capable of satisfactorily sterilizing the recombinant microorganism (*E. coli*) in low concentrations. The counting of viable cells was performed by the following procedures: the precipitate obtained by centrifuging 1 ml of the liquid culture for 5 minutes at 12,000 rpm was washed with a germ-free physiological saline three times, and thereafter, 1 ml of the same saline was added to the precipitate; 0.1 ml of the suspension or a suspension diluted appropriately with saline was coated onto a medium that was the same as what was used in cultivation except that the former contained 2% agar, and the number of colonies that formed as a result of cultivation for 24 hours at 37°C was counted. At least two countings were made for the same sample and the average thereof is shown in Table 1.

The effects of treatment with chlorhexidine gluconate on the activity (antiviral activity) of the polypeptide having the human interferon activity that was produced from the recombinant *E. coli* were investigated and the results are also shown in Table 1, from which one can see that within the range tested, the concentration of chlorhexidine gluconate did not have any substantial effect on the activity of the polypeptide (a small range of variations did occur but it would have resulted from the errors caused in the activity measurement by dilutions by different ratios). The procedures of antiviral activity measurement were as follows: the cells obtained by centrifuging the liquid culture were disrupted by freezing and thawing cycles and the supernatant was examined for its antiviral activity by the ordinary assay method.

TABLE 1

Effect (1) of the concentration of chlorhexidine gluconate on sterilization and antiviral activity

| Chlorhexidine gluconate (%) | No. of viable cells (CFU/ml) | Antiviral activity (units/ml) |
|---|---|---|
| 0 | $3.2 \times 10^7$ | $3.8 \times 10^5$ |
| 0.03 | $4.3 \times 10^6$ | $3.8 \times 10^5$ |
| 0.06 | $1.6 \times 10^4$ | $3.8 \times 10^5$ |
| 0.09 | $1.0 \times 10^3$ | $1.9 \times 10^5$ |
| 0.12 | 0 | $3.8 \times 10^5$ |
| 0.18 | 0 | $1.9 \times 10^5$ |

*E. coli* W3110/pIN5T4 was cultured under the same conditions as used above, whereby samples of liquid culture containing different numbers of viable cells were obtained. To these cultures, chlorhexidine gluconate was added in varying amounts ranging from 0.06% to 0.36%, and the number of viable cells in each of the treated cultures and their antiviral activity were determined by the same procedures as used above. The results are shown in Table 2, from which one can see that all of the recombinant *E. coli* cells were killed by using 0.18—0.36% of chlorhexidine gluconate, and that the activity of interferon produced by such *E. coli* cells was little affected by the chlorhexidine gluconate.

TABLE 2

Effect (2) of the concentration of chlorhexidine gluconate on sterilization and antiviral activity

| Chlorhexidine gluconate (%) | No. of viable cells (CFU/ml) | Antiviral activity (units/ml) |
|---|---|---|
| 0 | $7.5 \times 10^7$ | $3.8 \times 10^5$ |
| 0.06 | $2.3 \times 10^7$ | $1.9 \times 10^5$ |
| 0.12 | $4.5 \times 10^5$ | $2.9 \times 10^5$ |
| 0.18 | 0 | $3.8 \times 10^5$ |
| 0.24 | 0 | $3.8 \times 10^5$ |
| 0.36 | 0 | $3.8 \times 10^5$ |

Example 2

Sterilization of Mass-Cultured Recombinant Microorganism:

*E. coli* strain W3110/pIN5T4 (disclosed in Japanese Patent Application No. 132524/1983) capable of producing a polypeptide having human interferon activity was cultivated under aeration and agitation for 36 hours at 30°C in 700 liters of a medium containing 3% polypeptone, 2% yeast extract, 22% glucose, 0.5% $KH_2PO_4$, 0.01% $MgSO_4 \cdot 7H_2O$ and 20 µg/ml of tetracycline. The medium had been inoculated with a seed culture (500 ml) of about $1 \times 10^9$ *E. coli* cells/ml obtained by cultivation on a medium having the same composition as described above.

To 700 liters of the liquid culture of *E. coli* was added a 20% aqueous solution of chlorhexidine gluconate in an amount of 0.15% in terms of chlorhexidine gluconate. After agitation at 30°C for 1 hour, the number of viable cells in the culture was counted by the same method as described in Example 1 (the culture prior to treatment with chlorhexidine gluconate contained $5 \times 10^8$ CFU/ml). No viable cell was found in the treated culture.

A 10-ml sample was taken from each of the untreated culture and the culture treated by the method of the present invention. Each sample was centrifuged to collect the cells which were disrupted by freezing and thawing cycles, and the antiviral activity of the interferon in the supernatant was determined by a routine method. Both samples were found to have the same specific interferon activity. An extract was obtained from each of the cultured cells and the two extracts provided the same pattern of protein bands in SDS polyacrylamide gel electrophoresis (SDS-PAGE). It was therefore confirmed that even mass-cultured *E. coli* cells could be sterilized with chlorhexidine gluconate without causing any damage to the desired protein having interferon activity.

As will be shown in the following Reference Example, a pure polypeptide having human interferon activity could be obtained from the cultured cells that were sterilized by the aforementioned method of the present invention. The pure polypeptide sample was found to be entirely free from the chlorhexidine gluconate by gas chromatography on Radial®-PAK C18 column.

Reference Example

Wet cells (800 g) were collected from a liquid culture that was sterilized by the same method as used in Example 2. From these cells, a pure form of polypeptide having human interferon activity was obtained by the following procedures.

The cells were suspended in 5.1 liters of cooled 20 mM tri-HCl buffer (hereinunder abbreviated as THB; pH, 7.4) containing 1 mM $ZnCl_2$. The suspension was homogenized with an ice-cooled homogenizer M15 and centrifuged for 20 minutes at 7,000 rpm. To the resulting supernatant, an aqueous solution of 15% polyethylene imine (PEI) that had been adjusted to a pH of 8.0 with HCl was added to give a final concentration of 0.75%. After stirring for 10 minutes, the mixture was left to stand at 4°C for 2 hours. The resulting precipitate was removed by centrifugation (7,000 rpm × 20 min.) to obtain 4.7 liters of a supernatant. This supernatant was equilibrated with 20 mM N-2-hydroxyethylpiperazine-N′-3-propane-sulfonic

acid buffer (EPPSP buffer) having a pH of 8.6. The equilibrated supernatant was loaded onto a QAE Sephadex® A—25 column to obtain fractions as the effluent. The fractions were subsequently loaded onto a CM Sepharose® CL 6B column equilibrated with 20 mM THB (pH, 7.4) containing 0.1% 2-mercaptoethanol (hereunder 2-ME). The adsorbed active fractions were eluted by a linear density gradient of 0—0.5 M NaCl so as to collect fractions that were found to have high interferon activity by the method described in Unexamined Published Japanese Patent Application No. 201959/1983. These fractions were salted out by addition of ammonium sulfate to provide 50% saturation, and thereafter they were centrifuged for 20 minutes at 7,000 rpm to obtain a precipitate. The precipitate was dissolved in 20 mM sodium phosphate buffer (hereunder 20 mM PSB; pH, 7.4) containing 0.3 M NaCl and 0.1% 2-ME, and the solution was loaded onto a Sephacryl® S—200 equilibrated with the same 20 mM PSB, whereby a polypeptide corresponding to 298 mg of GIF146 (the specific activity of interferon: 1.6—1.7 × $10^6$ laboratory unit per mg of protein) was obtained as the final pure product. SDS-PAGE showed that this polypeptide had a purity of at least 99%; the SDS-PAGE band was in agreement with the band for the desired polypeptide.

## Claims

1. A method of sterilizing a recombinant microorganism by addition of chlorhexidine gluconate to a liquid culture of said recombinant microorganism before a physiologically active substance is extracted and purified from said culture.

2. A method according to claim 1 wherein the chlorhexidine gluconate is added to the liquid culture in an amount of 0.03 to 0.5%.

3. A method according to claim 1 wherein the recombinant microorganism is one capable of producing a physiologically active substance.

4. A method according to claim 3 wherein the physiologically active substance is a polypeptide having the activity of gamma interferon.

5. A method according to claim 3 or 4 wherein the recombinant microorganism capable of producing a physiologically active substance is *E. coli*.

## Patentansprüche

1. Verfahren zur Sterilisierung eines rekombinanten Mikroorganismus durch Zugabe von Chlorhexidingluconat zu einer Flüssigkultur des genannten rekombinanten Mikroorganismus, und zwar bevor eine physiologisch aktive Substanz aus der genannten Kultur extrahiert und gereinigt wird.

2. Verfahren gemäß Anspruch 1, dadurch gekennzeichnet, daß das Chlorhexidingluconat der Flüssigkultur in einer Menge von 0,03 bis 0,5% zugefügt wird.

3. Verfahren gemäß Anspruch 1, dadurch gekennzeichnet, daß der rekombinante Mikroorganismus ein Mikroorganismus ist, der befähigt ist, eine physiologisch aktive Substanz zu erzeugen.

4. Verfahren gemäß Anspruch 3, dadurch gekennzeichnet, daß die physiologisch aktive Substanz ein Polypeptid mit der Wirksamkeit von gamma-Interferon ist.

5. Verfahren gemäß Anspruch 3 oder 4, dadurch gekennzeichnet, daß der rekombinante Mikroorganismus, der zur Erzeugung einer physiologisch aktiven Substanz befähigt ist, E. coli ist.

## Revendications

1. Procédé de stérilisation d'un microorganisme recombinant par addition de gluconate de chlorhexidine à une culture liquide dudit microorganisme recombinant avant qu'une substance physiologiquement active ne soit extraite et purifiée à partir de ladite culture.

2. Procédé selon la revendication 1, dans lequel le gluconate de chlorhexidine est ajouté à la culture liquide en une quantité de 0,03 à 0,5%.

3. Procédé selon la revendication 1, dans lequel le microorganisme recombinant est capable de produire une substance physiologiquement active.

4. Procédé selon la revendication 3, dans lequel la substance physiologiquement active est un polypeptide ayant l'activité du gamma-interféron.

5. Procédé selon la revendication 3 ou 4, dans lequel le microorganisme recombinant capable de produire une substance physiologiquement active est *E. coli*.